# EUROPEAN PATENT SPECIFICATION

(11) **EP 0 842 160 B1**
(45) Date of publication and mention of the grant of the patent: **14.11.2001**
(21) Application number: 96926793.9
(22) Date of filing: 26.07.1996
(51) Int. Cl.: C07D 251/24, C08J 3/24, C08K 5/3492

(54) **FLUORINATED ALKENYLTRIAZINES AND THEIR USE AS CROSSLINKING AGENTS**
FLUORINIERTE ALKENYLTRIAZINE UND IHRE VERWENDUNG ALS VERNETZUNGSMITTEL
TRIAZINES D'ALCOYLENE FLUOREES ET LEUR UTILISATION EN TANT QU'AGENTS DE RETICULATION

(30) Priority: 26.07.1995 US 1449 P
(43) Date of publication of application: 20.05.1998
(73) Proprietor: E.I. DU PONT DE NEMOURS AND COMPANY, Wilmington Delaware 19898 (US)
(72) Inventor: HUNG, Ming-Hong, Wilmington, DE 19803-2202 (US); LOGOTHETIS, Anestis, Leonidas, Wilmington, DE 19810-3430 (US); YANG, Zhen-Yu, Wilmington, DE 19810-2403 (US)
(74) Representative: Kuhnen & Wacker
(86) International application number: US9612384
(87) International publication number: WO9705122

(56) References cited:
- US-A- 3 532 696
- US-A- 3 654 273
- US-A- 3 810 874
- US-A- 3 847 916
- US-A- 4 035 565
- US-A- 4 320 216
- 'ULLMANN'S ENCYCLOPEDIA OF INDUSTRIAL CHEMISTRY' vol. A23, 1993, VCH VERLAGSGESELLSCHAFT MBH, WEINHEIM pages 267-268
- D. APOTHEKER: 'CURING OF FLUOROELASTOMERS BY PEROXIDES' RUBBER CHEM. TECHNOL. vol. 56, 1982, pages 1013 - 1018

## Description

This invention concerns selected fluorinated alkenyltriazines, and their use as crosslinking agents for fluorinated elastomers.

### TECHNICAL BACKGROUND

Fluorinated elastomers are items of commerce, being used for a variety of applications where chemical and/or thermal resistance is important. They are especially useful for a variety of seals, such as O-rings and chevron rings. These elastomers are normally crosslinked when formed into their final part shapes, and it is desirable that the crosslinks formed have at least as much chemical and thermal stability as the elastomeric polymer itself.

One method of forming crosslinks with polymers which have certain functional groups attached is the free radical "grafting" of certain polyolefins, see for instance U.S. Patents 4,320,216, 4,303,761, 4,299,958 and 4,035,565, which are all hereby included by reference. The alkenyl triazines described herein give vulcanizates with good properties and have good curing characteristics, such as fast curing but good scorch resistance. US-A-3 847 916 and US-A-3 532 696 disclose fluorocarbon triazines. US-A-3 654 273 discloses high molecular weight fluorinated alkyl trisubstitued triazines. US-A-3 810 874 discloses polymers prepared from poly(perfluoroalkylene oxide) compounds.

### SUMMARY OF THE INVENTION

This invention concerns a compound of the formula 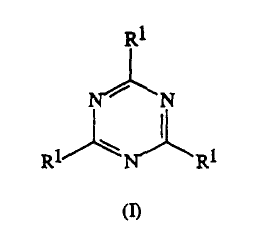 wherein R¹ is CH₂=CH(CF₂)ₙ-, CH₂=CHCH₂(CF₂)ₙ-, CH₂=CHCF₂CF(CF₃)OCF₂CF₂- or CH₂=CHCF₂CF₂CF(CH=CH₂)OCF₂CF(CF₃)OCF₂CF₂-, and n is an integer of 1 to 10.

This invention also concerns a process for the crosslinking of a fluoroelastomer, comprising, contacting a free radical generator, a fluoroelastomer which is a polymer containing fluorine whore glass transition temperature and melting point is at or below 40°C, and which contains 45% or more by weight or fluorine and a compound of the formula 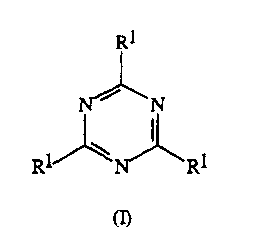 wherein R¹ is CH₂=CH(CF₂)ₙ-, CH₂=CHCH₂(CF₂)ₙ-, CH₂=CHCF₂CF(CF₃)OCF₂CF₂- or CH₂=CHCF₂CF₂CF(CH=CH₂)OCF₂CF(CF₃)OCF₂CF₂-, and n is an integer of 1 to 10, and provided said contacting is done at a temperature at which said free radical generator generates free radicals.

### DETAILS OF THE INVENTION

By a fluoroelastomer herein is meant a polymer containing fluorine whose glass transition temperature and melting point (if any) is at or below about 40°C. It is preferred that the fluoroelastomer contain about 45% or more by weight of fluorine, and more preferred that it is a perfluoroelastomer.

Compound (I) can generally be made by the trimerization of a nitrile of the formula R¹CN (see Examples 9, 12, 15, 20 and 25). These nitriles, and precursors thereto, can be made by methods illustrated in the Examples or found in the following references: G. A. Grindahl, et al., J. Org. Chem., vol. 32, pp. 603-607 (1967); and P. B. Sargent, et al., J. Am. Chem. Soc., vol. 91, p. 415ff (1969).

In compound (I), when R¹ is CH₂=CH(CF₂)ₙ-, it is preferred that n is 1 or 2, when R¹ is CH₂=CHCH₂(CF₂)ₙ-, it is preferred that n is 1.

When (I) is used as a crosslinking agent, it may be used to crosslink fluororelastomers made from the following monomer combinations: hexafluoropropylene/vinylidene fluoride; tetrafluoroethylene/vinylidene fluoride/hexafluoropropylene; tetrafluoroethylene/perfluoro(alkyl vinyl ether) wherein the alkyl group contains 1 to 5 carbon atoms, preferably wherein the alkyl group is methyl or propyl; and tetrafluoroethylene/perfluoro(alkyl vinyl ether) wherein the alkyl group contains one or more ether oxygen atoms and 2 to 20 carbon atoms. In all of the these polymers, 0.1 to 5 mole percent (based on total repeat units) of a repeat unit derived from a curesite monomer may optionally be present. A curesite monomer is a monomer which provides a repeat unit which aids in the crosslinkng process. A crosslinked polymer wherein (I) is used as a crosslinking agent is also novel, since the crosslink itself has not been included in such polymers.

The crosslinked polymers of this invention are useful wherever chemical and/or high temperature resistance is required. They are especially useful in sealing applications requiring such properties, such as in O-rings, chevron rings, gaskets, etc.

In the Examples, the following abbreviations are used:
Krytox® 16350 - poly(hexafluoropropylene oxide) available from E. I. du Pont de Nemours and Company, Wilmington, DE, USA
Luperco® 101XL - 2,5-dimethyl-2,5-bis(t-butylperoxy)hexane
PCN42 - a postcure cycle under nitrogen of 6 h at 90°C, 10 h ramp from 90 to 304°C, and 26 h at 304°C
PCN260 - a postcure cycle under nitrogen of 8 h ramp to 260°C, and then 40 h at 260°C
TAIC - triallyl isocyanurate

In Examples 25-27, numbers such as DXXXX refer to ASTM test methods for the tests performed. Abbreviations used herein to give the test results are given in the ASTM test methods.

### EXAMPLE 1

### Preparation of c-C₃F₅OCF₂CF(CF₃)OCF₂CF₂CO₂CH₃

### A 1 L autoclave was charged with 425 g of

CF₂-CFOCF₂CP(CF₃)OCF₂CF₂CO₂CH₃ and 335 g of hexafluoropropylene oxide and heated at 185°C for 10 hrs. The crude product (476.6 g) was distilled to give 391.6 g of pure product, bp 83-84°C/4.7 kPa. ¹⁹F NMR: -80.4 (s, 3F), -83.5 (m, 2F), -85.2 to -86.4 (m, 2F), -121.6 (s, 2F), -145.7 (t, J = 22 Hz, 1F), -152.9 9d, J = 193.4 Hz, 2F), -155.7 (dm, J = 194 Hz, 2F), -162.4 (t, J = 8.7 Hz, 1F). ¹H NMR: 3.97 (s). IR(neat): 1791 (s), 1308 (s), 1276 (s), 1239 (s), 1152 (s). Anal: calcd for C₁₀H₃F₁₅O₄: C, 25.44; H, 0.64. Found: C, 26.19; H, 0.73.

### EXAMPLE 2

### Reaction of c-C₃F₇OCF₂CF(CF₃)OCF₂CF₂CO₂CH₃ with Iodine

### A 1 L autoclave was charged with 200 g of

c-C₃F₇OCF₂CF(CF₃)OCF₂CF₂CO₂CH₃ (c = cyclo) and 108 g of I₂ and heated at 150°C for 5 hr. The product was washed with aqueous Na₂SO₃ solution, checked by GC, indicating 90% of product with 10% of starting material, and distilled to give 236.5 g of pure ICF₂CF₂CFIOCF₂CF(CF₃)OCF₂CF₂CO₂CH₃,
bp 107-110°C/399 Pa, and 21.6 g of bp 60-106°C/399 Pa material containing starting material. ¹⁹F NMR: -55.2 (d, J = 205.1 Hz, 1F), -58.8 (dm, J = 204.4 Hz, 1F), -69.0 (m, 1F), -80.0 (s, 3F), -79.6 to -80.7 (m, 1F). -82.5 to -84.0 (m, 2F), -89.9 (m, 0.5 F), -90.3 (m, 0.5F), -102.1 (d, J = 277.1 Hz, IF), -104.6 (dt, J = 277 Hz, J = 8.4 Hz, 1F), -121.5 (s, 2F), -145.7 (t, J = 11.3 Hz, 0.5F), -146.0 (t, J = 11.7 Hz, 0.5F). ¹H NMR: IR(neat): 2990 (w), 1786 (s), 1306 (s), 1243 (s), 1194 (s), 1152 (s), 1134 (s0, 1128 (s). Anal: Calcd for C₁₀H₃F₁₅I₂O₄: C, 16.55: H, 0.42; I, 34.96. Found: C, 17.03; H, 0.51; I, 35.21.

### EXAMPLE 3

### Reaction of c-C₃F₅OCF2CF(CF₃)OCF₂CF₂CO₂CH₃ with Iodine at Higher Temperature

A 0.4 L shaker tube was charged with 189 g of c-C₃F₅OCF₂CF(CF₃)OCF₂CF₂CO₂CH₃ and 100 g of I₂ and heated at 150°C for 3 hrs and 240°C for 8 hrs. Distillation of the reaction mixture gave 78.3 g of ICF₂CF₂COF, bp 57-58°C and 129.3 g of ICF₂CF(CF₃)OCF₂CF₂CO₂Me, bp 98-100°C/8.0 kPa. ¹⁹F NMR for ICF₂CF₂COF: +28.0 (m, 1F), -62.1 (m, 2F), -111.4 (m, 2F); for ICF₂CF(CF₃)OCF₂CF₂CO₂Me: -58.8 (dm, J = 210 Hz, 1F), -59.9 (dm, J = 210 Hz, IF), -76.8 (m, 3F), -82.7 (dm, J = 158.7 Hz, 1F), -83.7 (dm, J = 158 Hz, 1F), -121.6 (t, J = 3.3 Hz, 2F), -134.3 (m, 1F). IR for ICF₂CF₂COF: 1768 (s), 1187 (s), 1150 (s); IR for ICF₂CF(CF₃)CF₂CF₂CO₂Me: 1768 (s), 1342 9s), 1304 (s), 1232 to 1110 (s). Anal: Calcd. for C₇H₃F₁₀IO₃: C, 18.60; H, 0.67; F, 42.38; I, 28.08. Found: C, 18.24; H, 0.52; F, 42.38; I, 29.46.

### EXAMPLE 4

### Preparation of Ethyl 2-Iodotetrafluoropropanoate

A 300 mL shaker tube was charged with 50.8 g iodine and 50 g of trifluoromethoxylpentafluorocyclopropane and heated at 150°C for 4 hrs and 240°C for 8 hrs. After the tube was cooled to room temperature, 57.6 g of crude product was obtained, which was treated with 75 mL of EtOH and 11 g of KF at 10°C for 4 hours. The reaction mixture was poured into water. The lower layer was separated, washed with Na₂SO₃ solution and dried over molecular sieves to give 51.2 g of crude ester. Distillation gave 45.3 g of pure product,
bp 72-73°C/4.0 kPa. ¹H NMR: 4.43 (q, J = 7.0 Hz, 2H), 1.39 (t, J = 7.2 Hz, 3H). ¹⁹F NMR: -60.6 (t, J = 7.0 Hz, 2F), -111.9 (t, J = 7.0 Hz, 2F). IR (neat): 2995 (w), 1778 (s), 1374 (m), 1709 (s), 1185 (s), 1141 (s), 1076 (s). Anal: Calcd for C₅H₅F₄IO₂: C, 20.02; H, 1.68; F, 25.33; I, 42.30. Found: C, 19.83; H, 1.52; F, 27.74; I, 43.46.

### EXAMPLE 5

### Reaction of ICF₂CF₂CO₂Et with Ethylene

A 0.4 L shaker tube was charged with 100 g of ICF₂CF₂O₂Et, 0.5 g of limonene and 20 g of ethylene and heated at 210°C for 6 hours. Distillation of the reaction mixture gave 85 g of pure product, bp 83-84°C/665 Pa and 11 g of 84% pure product, bp 35-84°C/665 Pa. ¹⁹F NMR: -115.9 (t, J = 17.2 Hz, 2F), -120.4 (s, 2F). ¹H NMR: 4.41 (q, J = 7.1 Hz, 2H), 3.23 (m, 2H), 2.75 (m, 2H), 1.38 (t, J = 7.1 Hz, 3H). IR (neat): 2995 (w), 1774 (s), 1320 (s), 1167 (s), 1134 (s), 1113 (s). Anal: Calcd for C₇H₉F₄IO₂: C, 25.63; H, 2.77; F, 23.17; I, 38.68. Found: C, 26.50; H, 2.86; F, 25.38; I, 39.38.

### EXAMPLE 6

### Preparation of CH₂=CHCF₂CF₂CO₂Et

To a stirred solution of 705.2 g of ICH₂CH₂CF₂CF₂CO₂Et and 1 L of CH₂Cl₂ was slowly added 353 g of DBU over 3 hrs at 23 to 30°C. After the addition was complete, the reaction mixture was stirred at room temperature for 20 min. and then neutralized with 5% HCl solution. The organic layer was separated and the aqueous layer was extracted with CH₂Cl₂. The combined organic layers were washed with water and NaCl solution and dried over MgSO₄. After removal of CH₂Cl₂, the residue was distilled to give 331.5 g of CH₂=CHCF₂CF₂CO₂Et, bp 75°C/16 kPa.

### EXAMPLE 7

### Preparation of CH₂=CHCF₂CF₂CONH₂

To a stirred solution of 305 g (1.525 mol) of CH₂=CHCF₂CF₂CO₂Et and 700 mL of CH₂Cl₂ was added 34 g (2.0 mol) of NH₃ at 0°C over 1.5 hrs. After the addition was complete, the resulting mixture was stirred at room temperature overnight. Removal of all volatiles gave 221.3 g of white solid product. ¹⁹F NMR: -115.5 (2F), -122.0 (2F). ¹H NMR: 6.91 (br, 1H), 6.43 (br, 1H), 6.10-5.75 (m, 3H). IR(KBr): 3376 (m), 3268 (m), 3192 (m), 1706 (s), 1629 (m), 1245 (s), 1147 (s), 1014 (s), 956 (s).

### EXAMPLE 8

### Preparation of CH₂=CHCF₂CF₂CN

A mixture of 100 g of fine powder CH₂=CHCF₂CF₂CONH₂ and 261 g of P₂O₅ was heated at 130 to 170°C, during which volatiles were distilled out and collected in an ice-water cooled receiver. After 5 hours, 84.1 g of volatiles were obtained and GC analysis indicated the product was 97% pure. Two runs were combined, a drop of Hg added(to remove pink color), and distilled to give 160.7 g of colorless product, yield 90%, bp 53°C. ¹⁹F NMR: -107.5 (t, J = 4.3 Hz, 2F), -11.4 (t, J=4.3Hz, 2F). ¹H NMR: 6.10 to 5.90 (m).

### EXAMPLE 9

### Trimerzation of CH₂=CHCF₂CF₂CN

A 100 mL tube was charged with 40.0 g of CH₂=CHCF₂CF₂CN, 0.85 g of Ag₂O and cooled in liquid nitrogen. After being evacuated and pressured with nitrogen for six times, the tube was sealed and the contents in the tube were stirred at 120°C for 40 hours. The solids were dissolved in CH₂Cl₂ and transferred to a column with silica gel (CH₂Cl₂ as solvent) to give 35.0 g of pure (CH₂=CHCF₂CF₂)₃C₃N₃. ¹⁹F NMR: -113.7 (d, J = 11.1 Hz, 6F), -118.1 (s, 6F). IR: 1651 (w), 1552 (s), 1186-1014 (s). Anal: Calcd. for C₁₅H₉F₁₂N₃: C, 39.23; H, 1.98; N, 9.15. Found: C, 39.05; H, 1.94; N, 8.91.

### EXAMPLE 10

### Preparation of CH₂=CHCF₂CF(CF₃)OCF₂CF₂CO₂Me

A mixture of 68.0 g of ICF₂CF(CF₃)OCF₂CF₂CO₂Me and 8.0 g of CH₂=CH₂ was heated in a 0.1 L shaker tube at 210°C for 6 hours, and 63 g of crude product was obtained. The crude product was diluted with 100 mL of CH₂Cl₂ and treated with 25 g of DBU at room temperature overnight. The reaction mixture was poured to water and neutralized with a 5% HCl solution. The lower layer was separated and washed with water. After removal of the CH₂Cl₂, the residue was distilled to give 40.1 g (76%) of CH₂=CHCF₂CF(CF₃)OCF₂CF₂CO₂Me, bp 71-72°C/2.7 kPa. ¹⁹F NMR: -79.1 (m, 3F), -82.6 to -84.3 (m, 2F), -113.7 (dm J = 264.8 Hz, 1F), -115.0 (dm, J = 264.3 Hz, IF), -121.9 (t, J = 3.0 Hz, 2F), -145.3 (t, J = 21.8 Hz, IF). ¹H NMR: 3.96 (s, 3H), 5.99-5.78 (m, 3H).

### EXAMPLE 11

### Preparation of CH₂=CHCF₂CF(CF₃)OCF₂CF₂CN

A mixture of 21.1 g of CH₂=CHCF₂CF(CF₃)OCF₂CF₂CO₂Me and 12.0 g of NH₄OH (30% in H₂O) in 25 mL of acetone was stirred at room temperature overnight. After removal of all volatiles, 17.3 g of crude CH₂=CHCF₂CF(CF₃)OCF₂CF₂CONH₂ was obtained. ¹⁹F NMR: -79.1 (m, 3F), -82.5 (dd, J = 138.2 Hz, J = 24.0 Hz, 1F), -83.9 (dm, J = 138 Hz, 1F), -113.7 (dm, J = 264 Hz, 1F), -114.8 (dm, J = 264 Hz, 1F), -123.2 (m, 2F), -145.3 (m, 1F). ¹H NMR: 7.08 (br, 1H), 6.53 (br, 1H), 5.76-5.98 (m, 3H).

A flask fitted with a distillation head was charged with 13.0 g of the above amide and 18.0 g of P₂O₅ and was heated at 150 to 200°C for 2 hours, during which 9.3 g of CH₂=CHCF₂CF(CF₃)OCF₂CF₂CN was collected in a receiver, bp 103 to 104°C, 97.5% purity. ¹⁹F NMR: -79.1 (m, 3F), -83.6 (dm, J = 136 Hz, 1F), -85.3 (dm J = 136.8 Hz, 1F), -108.9 (m, 2F), -113.4 (dm, J = 264.8 Hz, 1F), -115.0 (dm, J = 265 Hz, 1F), -144.8 (m, 1F). ¹H NMR: 5.84 to 6.06 (m, 3H). Anal: Calcd for C₈H₃F₁₀NO: C, 30.11; H, 0.95; F, 59.54; N, 4.39. Found: C, 30.61; H, 1.17.

### EXAMPLE 12

### Trimerization of CH₂=CHCF₂CF(CF₃)OCF₂CF₂CN

A mixture was 4.0 g of CH₂=CHCF₂CF(CF₃)OCF₂CF₂CN and 0.12 g of Ag₂O was stirred at 140 to 150°C for 15 hours and then purified by chromatography on silica gel using a mixture of hexane and ethyl acetate in a 90 to 10 ratio as eluent to give 3.5 g of pure [CH₂=CHCF₂CF(CF₃)OCF₂CF₂]₃C₃N₃. ¹⁹F NMR: -79.4 (m, 9F), -82.30 (m, 6F), -113.8 (dm, J = 265 Hz, 3F), -114.6 (dm, J = 265 Hz, 3F), -119.4 (m, 6F), -144.8 (m, 3F). ¹HNMR: 5.98-5.75 (m, 9H). IR: 1651 (m), 1554 (s), 1423 (s), 1316 (s), 1219 to 1027 (s), 980 (s). Anal: Calcd for C₂₄H₉F₃₀N₃O₃: C, 30.11; H, 0.95; F, 59.54; N, 4.39. Found: C, 29.86; H, 1.02; F, 60.53; N, 4.45.

### EXAMPLE 13

### Preparation of CH₂=CHCF₂CF₂CF(CH=CH)₂OCF₂CF(CF₃)OCF₂CF₂CO₂Me

A mixture of 70.0 g of ICF₂CF₂CFIOCF₂CF(CF₃)OCF₂CF₂CO₂Me and 12.0 g of CH₂=CH₂ was heated in a 0.1 L shaker tube at 160°C for 3 hours and 190°C for 2 hours, and 65 g of crude product was obtained. The crude product was diluted with 60 mL of CH₂Cl₂ and treated with 33.4 g of DBU at room temperature for 3 hours. The reaction mixture was poured to water and neutralized with a 5% HCl solution. The lower layer was separated and washed water. After removal of the CH₂Cl₂, the residue was distilled to give 28.3 g (58%) of CH₂=CHCF₂CF₂CF(CH=CH₂)OCF₂CF(CF₃)OCF₂CF₂CO₂Me,
bp 108-110°C/665 Pa. ¹⁹F NMR: -78.5 to -79.4 (m, 1F), -80.2 (m, 3F), -82.5 (m, 1F), -83.1 (dm, J = 138.5 Hz, 1F), -84.2 (dm, J = 138.5 Hz, 1F), -112.8 (s, 2F), -121.8 (m, 2F), -124.6 (dd, J = 282.7 Hz, J = 24.8 Hz, IF), -125.3 (dm, J = 282.7 Hz, IF), -127.8 (m, IF), -145.8 (m, 1F). ¹H NMR: 3.95 (s, 3H), 5.10-5.69 (m, 6H). Anal: Calcd. for C₁₄H₉F₁₅O₄: C, 31.96; H, 1.72. Found: C, 31.98; H, 1.72.

### EXAMPLE 14

### Preparation of CH₂=CHCF₂CF₂CF(CH=CH₂)OCF₂CF(CF₃)OCF₂CF₂CN

A mixture of 20.0 g of CH₂=CHCF₂CF₂CF(CH=CH₂)OCF₂CF(CF₃)OCF₂CF₂CO₂Me and 3.0 g of NH₃ in 40 mL of CH₂Cl₂ was stirred at room temperature for 20 hours. After removal of all volatiles, 19.0 g of crude CH₂=CHCF₂CF₂CF(CH=CH₂)OCF₂CF(CF₃)OCF₂CF₂CONH₂ was obtained. ¹H NMR: 8.11 (br, 1H), 7.80 (br, 1H), 6.34-5.84 (m, 6H).

A flask fitted with a distillation head was charged with 17.0 g of above amide and 18.0 g of P₂O₅ and was heated at 160 to 200°C for 2.5 hours, during which 14.7 g of CH₂=CHCF₂CF₂CF(CH=CH₂)OCF₂CF(CF₃)OCF₂CF₂CN was collected in a receiver, bp 103 to 104°C. ¹⁹F NMR: -78.1 to -79.0 (m, IF), -80.3 (m, 3F), -81.6 to -82.4 (m, 1F), -84.3 (dm J = 134.8 Hz, IF), -85.2 (dm J = 134.8 Hz, 1F), -108.8 (m, 2F), -112.8 (dm, 2F), -124.4 (ddd. J = 283.8 Hz, J = 28 Hz, J = 4 Hz, 1F), -125.6 (dd, J = 282.6 Hz, J = 10.0 Hz, IF), -127.8.8 (m, 1F), -145.2 (m, 1F). ¹H NMR: 5.70-6.20 (m). Anal: Calcd. for C₁₃H₆F₁₅NO₂: C, 31.66; H, 1.23; N, 2.84. Found: C, 31.73; H, 1.40; N, 3.10.

### EXAMPLE 15

### Trimerization of CH₂=CHCF₂CF₂CF(CH=CH₂)OCF₂CF(CF₃)OCF₂CF₂CN

A mixture of 2.0 g CH₂=CHCF₂CF₂CF(CH=CH₂)OCF₂CF(CF₃)OCF₂CF₂CN and 0.1 g of NH₃ was heated at 140°C in a sealed tube for 36 hours. The viscous oil was diluted with CH₂Cl₂ and transferred to a flask. After removal of solvent, the residue was purified by chromatography using ethyl acetate and hexane (9:1) as solvent to give 1.6 g of product. ¹⁹F NMR: -78.3 to -78.9 (m, 3F), -80.5 (m, 9F), -81.9 to -83.3 (m, 3F), -113.1 (d, J = 7.3 Hz, 6F), -119.5 (m, 6F), -124.6 (dd, J = 283.6 Hz, J = 18.3 Hz, 3F), -126.0 (dd, J = 282.5 Hz, J = 18.2 Hz, 3F), -127.9 (m, 3F), -145.6 (m, 3F). IR: 3101 (w), 1735 (w), -1651 (w), 1555 (s), 1238-1008 (s). Anal: Calcd. for C₃₉H₁₈F₄₅N₃O₆: C, 31.66; H, 1.23; F, 57.78; N, 2.84. Found: C, 31.03; H, 1.45; N, 2.67.

### EXAMPLE 16

### Preparation of Ethyl 4-Iodo-2,2-difluorobutyrate

In a one-liter pressure reactor was charged ethyl iododifluoroacetate (200 g, 0.8 mol, from accompanied patent proposal), CH₃CN (80 mL) and water (300 mL). The mixture was cooled to -10°C and then a mixture of Na₂S₂O₄ (40 g) and NaHCO₃ (20 g) was added. The reactor was closed, cooled evacuated and charged with ethylene (60 g, 2.14 mol). The reaction mixture was then warmed to room temperature in a 4 hr period and kept at 40°C for 2 hr. After the reaction was over the lower layer was separated from the reaction mixture and the aqueous layer was extracted with ether. The combined organic layer was washed with brine and dried over MgSO₄. Distillation gave the title compound (200 g, 90% yield), bp. 70°C/266 Pa. ¹H NMR (300 MHz, CDCl₃): d1.38 (t, 3H), 2.70 (m, 2H), 3.20 (m, 2H), 4.37 (q, 2H). ¹⁹F NMR (188.24 MHz, CDCl₃): -107.3 (t, J =16 Hz, 2F). Anal. Calcd. for C₆H₉F₂IO₂: C, 25.92; H, 3.26; F, 13.67. Found: C, 26.69; H, 3.28; F, 13.39. IR (neat): 1780 cm⁻¹ (C=O). Mass: Calcd. for [(M+H)⁺]: 278.9691; Found: 278.9659.

### EXAMPLE 17

### Preparation of Ethyl 2,2-Difluoro-3-butenoate

To a stirred solution of ICH₂CH₂CF₂CO₂Et (450 g, 1.62 mol) in ether (1000 mL) was added dropwise 1,8-diazabicyclo[5.4.0]undec-7-ene (DBU, 260 g, 1.71 mol) in a 2 hr period. The temperature was maintained at between 10-20°C with external cooling. After the addition was complete, the reaction mixture was stirred at room temperature for 4 hrs. Water (600 mL) was added and the ethereal layer was separated and washed with brine, dried over MgSO₄. Distillation gave the desired product (200 g, 82% yield), bp. 60°C/9.3 kPa. ¹H NMR (300 MHz, CDC13): d1.32 (t, 3H), 4.30 (q, 2H), 5.60 (d, 1H), 5.80 (dt, 1H), 6.00 (m, 1H). ¹⁹F NMR (188.24 MHz, CDCl₃): -106.2 (2F). Anal. Calcd. for C₆H₈F₂O₂: C, 48.00; H, 5.37; F, 25.31. Found: C, 47.82; H, 5.72; F, 27.32. IR (neat): 1770 cm⁻¹ (C=O), 1650 cm⁻¹ (C=C). Mass: Calcd. for [(M-CH₂=CH₂)⁺]: 122.0179; Found: 122.0191.

### EXAMPLE 18

### Preparation of 2,2-Difluoro-3-butenamide

Ethyl 2,2-difluoro-3-butenonate (51 g, 0.34 mol) was added dropwise into a solution of ammonium hydroxide (28-30 wt%, 24 g, 0.4 mol) and THF (25 mL) with stirring. The temperature was maintained at 10-20°C with external cooling during the addition. After addition was completed, the reaction mixture was stirred at ambient temperature for 3 hr. The disappearance of the starting material was monitored by GC. Extraction with ether followed by evaporation of solvent gave pure amide as a white crystal (30.5 g, 74%), mp. 85-86°C. ¹H NMR (300 MHz, acetone-d₆): d5.65 (m, 1H), 5.80 (m, 1H), 6.18 (m, 1H), 7.33 (br., 1H), 7.62 (br., 1H). ¹⁹F NMR (188.24 MHz, acetone-d₆): -104.9 (2F). Anal. Calcd. for C₄H₅F₂NO: C, 39.68; H, 4.16; N, 11.57. Found: C, 39.83; H, 4.01; N, 11.23. IR (KBr): 3200, 3380 cm⁻¹ (br, CONH₂), 1690 cm⁻¹ (C=O), 1650 cm⁻¹ (C=C). Mass: Calcd. for [(M-F)⁺]: 102.0355; Found: 102.0372.

### EXAMPLE 19

### Preparation of 2,2-Difluoro-3-butenenitrile

2,2-Difluoro-3-butenamide (14.0 g, 0.118 mol) was well mixed with P₂O₅ (10 g) and heated slowly to 200°C. The product was distilled at 42-43°C to give the nitrile product (10.8 g, 91% yield). ¹H NMR (300 MHz, CDCl₃): d5.80 (m, 1H), 6.05 (m, 2H). ¹⁹F NMR (188.24 MHz, CDCl₃): -86.5 (m, 2F). Mass: Calcd. for [M⁺]: 103.0233; Found: 103.0227.

### EXAMPLE 20

### Preparation of 2,4,6-Tris(1',1'-difluoroallyl)-1,3,5-triazine

A mixture of 2,2-difluoro-3-butenenitrile (18.5 g, 0.18 mol) and ammonia (ca. 0.1 g) was heated at 120-130°C for 3 hr and then distilled to give the desired triazine product (17.8 g, 96% yield), bp.70°C/80 Pa. ¹H NMR (300 MHz, CDCl₃): d5.73 (d, 3H), 5.98 (d, 3H), 6.32 (m, 3H). ¹⁹F NMR (188.24 MHz, CDCl₃): -103.6. IR (neat): 1554 cm⁻¹ (triazine), 1650 cm⁻¹ (C=C). Anal. Calcd. for C₁₂H₉F₆N₃: C, 46.61; H, 2.93; N, 13.59; F, 36.86. Found: C, 44.98; H, 3.13; N, 13.30; F, 38.08. Mass: Calcd. for [M⁺]: 309.0700; Found: 309.0695.

### EXAMPLE 21

### Preparation of Ethyl 2,2-Difluoro-4-pentenoate

Ethyl iododifluoroacetate (100 g, 0.4 mol) was added dropwise into a well stirred suspension of copper powder (51 g, 0.803 mol) in anhydrous DMSO (250 mL) at room temperature. The temperature was maintained at below 25°C during the addition with external cooling. After that, the reaction mixture was stirred at room temperature for 45 min. Allyl bromide (60.5 g, 0.5 mol) was then added dropwise while the temperature was still controlled at 20-25°C during the addition. The reaction mixture was stirred at room temperature for another 3 hr after the addition was completed. Distillation in vacuo gave a crude product, which was extracted with ether, washed with brine and dried over MgSO₄. Redistillation produced pure ethyl 2,2-difluoro-4-pentenoate (52.8 g, 80% yield) as a clear, colorless liquid, bp. 55°C/2.7 kPa. ¹H NMR (300 MHz, CDCl₃): d1.25 (t, 3H), 2.76 (dt, 2H), 4.24 (q, 2H), 5.20 (1H), 5.22 (1H), 5.63 (m, 1H). ¹⁹F NMR (188.24 MHz, CDCl₃): -105.7 (t, J = 18.9 Hz). IR (neat): 1780 cm⁻¹ (C=O), 1650 cm⁻¹ (C=C). Anal. Calcd. for C₇H₁₀F₂O₂: C, 51.22; H, 6.14; F, 23.15. Found: C, 49.73; H, 6.06; F, 21.42. Mass: Calcd. for [M⁺]: 164.0648; Found: 164.0645.

### EXAMPLE 22

### Preparation of 2,2-Difluoro-4-pentenamide

To a stirred solution of ammonium hydroxide (28-30 wt%, 50 mL) and THF (200 mL) was added dropwise CH₂=-CHCH₂CF₂CO₂Et (51 g, 0.31 mol). The temperature was maintained at 20-25°C (external cooling if necessary) during the addition. The reaction was complete within 3 hr as monitored by GC. The product was worked up to give the corresponding amide compound (36 g, 86% yield), bp. 75°C/93 Pa.
¹H NMR (300 MHz, acetone-d₆): d2.85 (dt, J = 7.2 Hz, J =16 Hz, 2H), 5.20-5.30 (m, 2H), 5.70-5.85 (m, 1H), 7.25 (br., 1H), 7.58 (br., 1H). ¹⁹F NMR (188.24 MHz, acetone-d₆): -105.3 (t, J =16 Hz, 2F). IR (neat): 3200-3500 cm⁻¹ (N-H), 1720 cm⁻¹ (C=O), 1650 cm⁻¹ (C=C). Anal. Calcd. for C₅H₇F₂NO: C, 44.45; H, 5.22; N, 10.37; F, 28.12. Found: C, 42.31; H, 5.22; N, 10.18; F, 29.93. Mass: Calcd. for [M⁺]: 135.0495; Found: 135.0490.

### EXAMPLE 23

### Preparation of 2,2-Difluoro-4-pentenenitrile

A mixture of CH₂=CHCH₂CF₂CONH₂ (33.8 g, 0.25 mol) and P₂O₅ (40 g) was heated slowly to 170-200°C to give the desired product as a colorless liquid (25.2 g, 86% yield), bp. 72-74°C. ¹H NMR (300 MHz, CDCl₃): d2.85 (dt, J = 7.2 Hz, J = 18 Hz, 2H), 5.30-5.50 (m, 2H), 5.62-5.78 (m, 1H). ¹⁹F NMR (188.24 MHz, CDCl₃): -89.5 (t, J =18 Hz). IR (gas): 2260 cm⁻¹ (C°N), 1650 cm⁻¹ (C=C). Mass: Calcd. for [M⁺]: 117.0390; Found: 117.0382.

### EXAMPLE 24

### Preparation of 2,4,6-Tris(1',1'-difluoro-3'-butenyl)-1,3,5-triazine

A mixture of 2,2-difluoro-4-pentenenitrile (23.4 g, 0.2 mol) and ammonia gas (ca. 0.1 g) was heated at 120°C for 10 hr in a sealed tube, and then distilled to give the desired triazine (18.5 g, 79% yield), bp. 86-90°C/93 Pa. ¹H NMR (300 MHz, CDCl₃): d3.15 (dt, J = 7.2 Hz, J = 16 Hz, 2H), 5.18-5.30 (m, 2H), 5.70-5.90 (m, 1H). ¹⁹F NMR (188.24 MHz, CDCl₃): -103.1 (t, J =16 Hz, 2F). IR (neat): 1555 cm⁻¹ (triazine), 1645 cm⁻¹ (C=C). Anal. Calcd. for C₁₅H₁₅F₆N₃: C, 51.29; H, 4.30, N, 11.96; F, 32.45. Found: C, 50.50; H, 4.28; N, 11.80; F, 31.22. Mass: Calcd. for [M⁺]: 351.1170; Found: 351.1164.

### EXAMPLE 25

A perfluoroelastomer was prepared in a continuous polymerization process, similar to that described in U.S. Patent 4,983,697. The polymer was prepared in a 2 L mechanically stirred, water jacketed, stainless-steel autoclave operated continuously at 90°C and 6.2 MPa into which was pumped at a rate of 550 ml/h an aqueous polymerization medium/initiator solution comprising of 16 liters of water, 62 g of ammonium persulfate, 337 g of disodium hydrogen phosphate heptahydrate, 220 g of ammonium pefluorooctanoate ("Fluorad" FC-143 from 3M Co.). At the same time a separate solution ofperfluoro(-(8-cyano-5-methyl-3,6-dioxa-1-octene) (8CNVE) was added at a rate of 7.4 g/h of 8CNVE. A gaseous stream of tetrafluoroethylene (113 g/h) and perfluoro(methyl vinyl ether) (PMVE, 130 g/h) were fed in the reactor at a constant rate by means of a diaphragm compressor. The polymer was continuously removed by means of a let-down valve and unreacted monomers were vented. The latex from 27.6 h of operation was combined and the polymer was coagulated by adding it with stirring to a hot (90-95°C) magnesium sulfate heptahydrate solution of about 3700 g in 80 L of water. The coagulated crumb was repeatedly washed with fresh water and dried at 80°C in an air oven. Analysis of the polymer by infrared indicated that the PMVE content was 44.6 wt%, TFE 53.1 wt% and 8CNVE 2.3 wt%. The inherent viscosity was 0.44 and the Mooney viscosity (ASTM D1646) was 32 as measured at 150°C and 86 as measured at 121°C.

The polymer was compounded on a rubber mill using the formulation shown in Table 1. The parts O-rings (size 214) and sheets were crosslinked by heating then in a hydraulic press at 175°C/30 min. under 3.45 MPa. They were then post-cured at 305°C for 42 hrs under nitrogen or at 225°C for 24 hr in air and tested using ASTM methods. Under column A we show the results of crosslinking the above polymer using just peroxide and a coagent, using triallyl isocyanurate as the control and comparative results with the trivinyl perfluoroalkyl triazines where n = 2 (column B) and n = 1 (column C). The properties of parts molded as O-ring and as dumbbells are being compared.

**TABLE 1**

| TRIS(VINYLTETRAFLUOROETHYLENE)TRIAZINE (TVTFET) AND TRIS(VINYLDIFULOROMETHYLENE)TRIAZINE (TVDFMT) AS COAGENTS IN THE PEROXIDE CURING OF PERFLUOROELASTOMER | | | |
|---|---|---|---|
| | A | B | C |
| | (TAIC) | (TVTFET) | (TVDFMT) |
| COMPOUND | | | |
| Polymer | 100 | 100 | 100 |
| MT Black | 30 | 30 | 30 |
| Krytox® 16350 | 10 | 10 | 10 |
| Luperco® 101XL | 3 | 3 | 3 |
| ZnO | 2 | 2 | 2 |
| TRIALLYL ISOCYANURATE | 3 | --- | ---- |
| TVTFET | --- | 3 | --- |
| TVDFMT | --- | --- | 3 |
| | | | |

| ODR 177°/3° Arc D2084 | | | |
|---|---|---|---|
| M1, Nm | 0.60 | 0.23 | 0.68 |
| ts₂, mins | 1.5 | 3.5 | 7.5 |
| MH, Nm | 2.5 | 1.7 | 1.8 |
| MH-ML, Nm | 1.9 | 1.5 | 1.1 |

| PROPERTIES | O-Rings¹ | Dumb Bells² | O-Rings¹ | Dumb Bells² | O-Rings¹ |
|---|---|---|---|---|---|
| Tensile D1708 | | | | | |
| M50, MPa | 5.07 | | 4.24 | | 4.05 |
| M100, MPa | 9.04 | 8.36 | 8.50 | 8.87 | 7.11 |
| Tb, MPa | 12.1 | 14.7 | 13.6 | 17.7 | 8.94 |
| Eb, % | 144 | 260 | 173 | 230 | 143 |
| Comp. Set 204°C/ 70 hr D1414 | 56 | | 70 | | 60 |

| | | | | | |
|---|---|---|---|---|---|
| ¹Post-cured at 305°C/42 h under nitrogen (PCN42) | | | | | |
| ²Post-cured at 225°C/24 hr in air | | | | | |

### EXAMPLE 26

The same polymer was used as described in Example 25. The formulation shown in Table 2 is based on the dual cure system which utilizes both the peroxide/coagent and the triphenyl tin hydroxide catalyst (TPT-OH).

**TABLE 2**

| TRIS(VINYLTETRAFLUOROETHYLENE)TRIAZINE (TVTFET) AND TRIS(VINYLDIFULOROMETHYLENE)TRIAZINE (TVDFMT) AS COAGENTS IN THE DUAL CURE OF PERFLUOROELASTOMER | | | |
|---|---|---|---|
| | A | B | C |
| | (TAIC) | (TVTFET) | (TVDFMT) |
| COMPOUND | | | |
| Polymer | 100 | 100 | 100 |
| MT Black | 30 | 30 | 30 |
| Krytox® 16350 | 10 | 10 | 10 |
| ZnO | 2 | 2 | 2 |
| TPT-OH | 1 | 1 | 1 |
| Luperco® 101XL | 1 | 1 | 1 |
| TAIC | 1 | ---- | ---- |
| TVTFETriazine | ---- | 1 | ---- |
| TVDFMTriazine | ---- | ---- | 3 |
| | | | |

| ODR 177°/3° Arc D2084 | | | |
|---|---|---|---|
| M1, Nm | 0.68 | 0.40 | 0.79 |
| ts₂, mins | 2.5 | 3.5 | 2.5 |
| MH, Nm | 4.6 | 3.2 | 3.1 |
| MH-ML, Nm | 4.0 | 2.8 | 2.3 |

| PROPERTIES | O-Rings¹ | Dumb Bells² | O-Rings¹ | Dumb Bells² | O-Rings¹ |
|---|---|---|---|---|---|
| Tensile D1708 | | | | | |
| M50, MPa | 4.24 | | 4.18 | 4.45 | |
| M100, MPa | 7.24 | 8.39 | 7.14 | 7.42 | 10.8 |
| Tb, MPa | 10.4 | 17.1 | 10.5 | 9.54 | 18.2 |
| Eb, % | 167 | 240 | 186 | 157 | 200 |
| Comp. Set 204°C/ 70 hr D1414 | 54 | | 58 | 60 | |

| | | | | | |
|---|---|---|---|---|---|
| ¹Post-cured at 305°C/42 h under nitrogen (PCN42) | | | | | |
| ²Post-cured at 225°C/24 hr in air | | | | | |

### EXAMPLE 29

Viton® GF (a copolymer of vinylidene fluoride, hexafluoropropylene, tetrafluoroethylene and bromotrifluorobutene available from E. I. du Pont de Nemours and Company, Wilmington, DE, U.S.A.) was used in this Example. The formulation described in Table 3 was used.

**TABLE 3**

| TRIS(VINYLTETRAFLUOROETHYLENE)TRIAZINE (TVTFET) AND TRIS(VINYLDIFULOROMETHYLENE)TRIAZINE (TVDFMT) AS COAGENTS IN THE PEROXIDE CURING OF VITON® GF | | | | |
|---|---|---|---|---|
| | A | B | C | D |
| | (TAIC) | (TVTFET) | (TVDFET) | (TVDFMT) |
| COMPOUND | | | | |
| Polymer | 100 | 100 | 100 | 100 |
| MT Black | 30 | 30 | 30 | 30 |
| MgO | 3 | 3 | 3 | 3 |
| Luperco® 101XL | 3 | 3 | 3 | 3 |
| TAIC | 3 | ---- | ---- | ---- |
| TVTFETriazine | ---- | 3 | 1.5 | ---- |
| TVDFMTriazine | ---- | ---- | ---- | 1.5 |
| | | | | |
| ODR 177°/3° Arc D2084 | | | | |
| Ml, Nm | 1.0 | 0.90 | 0.90 | 0.90 |
| ts₂, mins | 1.5 | 5.5 | 5.0 | 3.5 |
| MH, Nm | 4.3 | 2.4 | 2.6 | 2.4 |
| MH-ML, Nm | 3.3 | 1.5 | 1.7 | 1.5 |

| PROPERTIES (O-Rings¹) D1414 | | | | |
|---|---|---|---|---|
| M50, MPa | 2.95 | 2.34 | | 2.18 |
| M100, MPa | 8.06 | 4.83 | | 4.43 |
| Tb, MPa | 14.6 | 14.1 | | 11.7 |
| Eb,% | 143 | 215 | | 202 |
| | | | | |
| Comp. Set 204°C/ | 53 | 73 | 79 | 58 |
| 70 h D1414 | | | | |
| (Dumbbells²) D1708 | | | | |
| M100, MPa | 5.58 | 3.50 | 3.36 | 3.89 |
| Tb, MPa | 22.4 | 22.5 | 22.7 | 22.1 |
| Eb, % | 230 | 400 | 400 | 360 |

| | | | | |
|---|---|---|---|---|
| ¹Post-cured at 260°C/48 h under nitrogen (PCN260) | | | | |
| ²Post-cured at 225°C/24 h in air | | | | |

## Claims

1. A compound of the formula 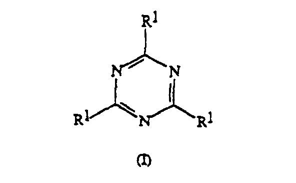 wherein R¹ is
CH₂=CH(CF₂)ₙ-,
CH₂=CHCH₂(CF₂)ₙ-,
CH₂=CHCF₂CF(CF₃)OCF₂CF₂-
or
CH₂=CHCF₂CF₂CF(CH=CH₂)OCF₂CF(CF₃)OCF₂CF₂-,
and n is an integer of 1 to 10.

2. The compound as recited in Claim 1 wherein when R¹ is CH₂=CH(CF₂)ₙ- and n is 1 or 2.

3. The compound as recited in Claim 1 wherein R¹ is CH₂=CHCH₂(CF₂)ₙ- and n is 1.

4. The compound as recited in Claim 1 wherein R¹ is CH₂=CHCF₂CF(CF₃)OCF₂CF₂- or CH₂=CHCF₂CF₂CF(CH=CH₂)OCF₂CF(CF₃)OCF₂CF₂-.

5. A process for the crosslinking of a fluoroelastomer, comprising, contacting a free radical generator, a fluoroelastomer which is a polymer containing fluorine whose glass transition temperature and melting point is at or below 40°C, and which contains 45 % or more by weight of fluorine, and a compound of the formula 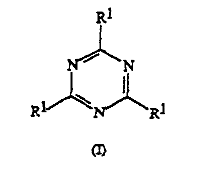 wherein R¹ and n are as defined in claim 1, and provided said contacting is done at a temperature at which said free radical generator generates free radicals.

6. The process as recited in Claim 5 wherein said fluoroelastomer is a copolymer of hexafluoropropylene/vinylidene fluoride; tetrafluoroethylene/vinylidene fluoride/hexafluoropropylene; tetrafluoroethylene/perfluoro(alkyl vinyl ether) wherein the alkyl group contains 1 to 5 carbon atoms, or and tetrafluoroethylene/perfluoro(alkyl vinyl ether) wherein the alkyl group contains one or more ether oxygen atoms and 2 to 20 carbon atoms.

7. The process as recited in Claim 5 wherein R¹ is CH₂=CH(CF₂)ₙ- and n is 1 or 2, or R¹ is CH₂=CHCH₂(CF₂)ₙ- and n is 1.

8. The product of the process of Claim 5.

9. The product of the process of Claim 6.

10. The process as recited in Claim 6 wherein said fluoroelastomer also contains 0.1 to 5 mole percent of a curesite monomer.

11. The product of the process of Claim 10.

12. The process as recited in Claim 5 wherein said fluoroelastomer contains 0.1 to 5 mole percent of a curesite monomer.

13. The process as recited in Claim 5 wherein said fluoroelastomer is a tetrafluoroethylene/perfluoro(alkyl vinyl ether) copolymer wherein the alkyl group is methyl or propyl.

14. The process as recited in Claim 13 wherein said fluoroelastomer contains 0.1 to 5 mole percent of a curesite monomer.

15. The product of the process of Claim 7.

16. The product of the process of claim 14.

## Patentansprüche

1. Verbindung der Formel 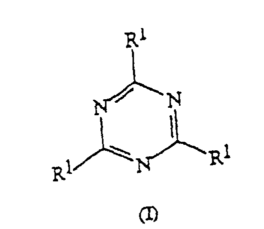 wobei R¹ für
CH₂=CH(CF₂)ₙ-,
CH₂=CHCH₂(CF₂)ₙ-,
CH₂=CHCF₂CF(CF₃)OCF₂CF₂-
oder
CH₂=CHCF₂CF₂CF(CH=CH₂)OCF₂CF(CF₃)OCF₂CF₂-
steht und n eine ganze Zahl von 1 bis 10 ist.

2. Verbindung nach Anspruch 1, wobei R¹ für CH₂=CH(CF₂)ₙ- steht und n für 1 oder 2 steht.

3. Verbindung nach Anspruch 1, wobei R' für CH₂=CHCH₂(CF₂)ₙ- steht und n für 1 steht.

4. Verbindung nach Anspruch 1, wobei R¹ für CH₂=CHCF₂CF(CF₃)OCF₂CF₂- oder CH₂=CHCF₂CE₂CF(CH=CH₂)OCF₂CF(CF₃)OCF₂CF₂- steht.

5. Verfahren zum Vernetzen eines Fluorelastomers, umfassend das Inkontaktbringen eines freie Radikale erzeugenden Mittels, eines Fluorelastomers, bei dem es sich um ein fluorhaltiges Polymer handelt, dessen Glasübergangstemperatur und Schmelzpunkt bei 40°C oder darunter liegt und das 45 Gew.-% oder mehr Fluor enthält, und einer Verbindung der Formel 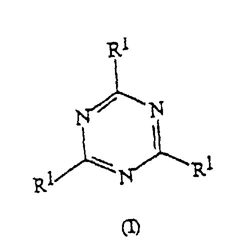 wobei R¹ und n die in Anspruch 1 angegebene Bedeutung besitzen, mit der Maßgabe, daß dieses Inkontaktbringen bei einer Temperatur ausgeführt wird, bei der dieses freie Radikale erzeugende Mittel freie Radikale erzeugt.

6. Verfahren nach Anspruch 5, wobei das Fluorelastomer ein Copolymer ist aus Hexafluorpropylen/Vinylidenfluorid; Tetrafluorethylen/Vinylidenfluorid/Hexafluorpropylen; Tetrafluorethylen/Perfluor(alkylvinylether), in dem die Alkylgruppe 1 bis 5 Kohlenstoffatome enthält, oder/und Tetrafluorethylen/Perfluor(alkylvinylether), in dem die Alkylgruppe ein oder mehrere Ethersauerstoff-Atome und 2 bis 20 Kohlenstoffatome enthält,.

7. Verfahren nach Anspruch 5, wobei R¹ für CH₂=CH(CF₂)ₙ- steht und n für 1 oder 2 steht, oder R¹ für CH₂=CHCH₂(CF₂)ₙ- steht und n für 1 steht.

8. Produkt des Verfahrens nach Anspruch 5.

9. Produkt des Verfahrens nach Anspruch 6.

10. Verfahren nach Anspruch 6, wobei das Fluorelastomer auch 0,1 bis 5 Mol% eines Curesitmonomers enthält.

11. Produkt des Verfahrens nach Anspruch 10.

12. Verfahren nach Anspruch 5, wobei das Fluorelastomer 0,1 bis 5 Mol% eines Curesitmonomers enthält.

13. Verfahren nach Anspruch 5, wobei das Fluorelastomer ein Tetrafluorethylen/Perfluor(alkylvinylether)-Copolymer ist, in dem die Alkylgruppe Methyl oder Propyl ist.

14. Verfahren nach Anspruch 13. wobei das Fluorelastomer 0,1 bis 5 Mol% eines Curesitmonomers enthält.

15. Produkt des Verfahrens nach Anspruch 7.

16. Produkt des Verfahrens nach Anspruch 14.

## Revendications

1. Composé de formule 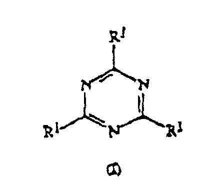 dans laquelle R¹ est
CH₂=CH(CF₂)ₙ-,
CH₂=CHCH₂(CF₂)ₙ-,
CH₂=CHCF₃CF(CF₃)OCF₂CF₂- ou
CH₂=CHCF₂CF₂CF(CH=CH₂)OCF₂CF(CF₃) OCF₂CF₂-,
et n est un nombre entier de 1 à 10.

2. Composé selon la revendication 1 dans lequel R¹ est CH₂=CH(CF₂)ₙ- et n est 1 ou 2.

3. Composé selon la revendication 1 dans lequel R¹ est CH₂=CHCH₂(CF₂)ₙ- et n est 1.

4. Composé selon la revendication 1 dans lequel R¹ est CH₂=CHCF₂CF(CF₃)OCF₂CF₂- ou
CH₂=CHCF₂CF₂CF(CH=CH₂) OCF₂CF (CF₃) OCF₂CF₂-.

5. Procédé pour la réticulation d'un fluoroélastomère, comprenant, la mise en contact d'un générateur de radicaux libres, d'un fluoroélastomère qui est un polymère contenant du fluor dont la température de transition vitreuse et le point de fusion est égal ou inférieur à 40°C, et qui contient 45% en poids ou davantage de fluor, et d'un composé de formule 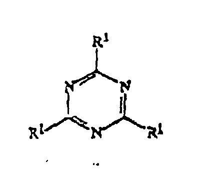 dans laquelle R¹ et n sont tels que définis à la revendication 1 et à condition que ladite mise en contact se fasse à une température à laquelle ledit générateur de radicaux libres génère des radicaux libres.

6. Procédé selon la revendication 5 dans lequel ledit fluoroélastomère est un copolymère d'un hexafluoropropylène et de fluorure de vinylidène ; un copolymère tétrafluoroéthylène, de fluorure de vinylidène et d'hexafluoropropylène ; un copolymère de tétrafluoroéthylène et de perfluoro(alkylvinyléther) dans lequel le groupement alkyle contient 1 à 5 atomes de carbone, ou et un copolymère de tétrafluoroéthylène et de perfluoro(alkylvinyléther) dans lequel le groupement alkyle contient un ou plusieurs atomes d'oxygène d'éther et 2 à 20 atomes de carbone.

7. Procédé selon la revendication 5 dans lequel R¹ est CH₂=CH(CF₂)ₙ- et n est 1 ou 2 ou R¹ est CH₂=CHCH₂(CF₂)ₙ- et n est 1.

8. Produit du procédé de la revendication 5.

9. Produit du procédé de la revendication 6.

10. Procédé selon la revendication 6 dans lequel ledit fluoroélastomère contient aussi 0,1 à 5% en mole d'un monomère faisant office d'emplacement de durcissement.

11. Produit du procédé de la revendication 10.

12. Procédé selon la revendication 5 dans lequel ledit fluoroélastomère contient 0,1 à 5% en mole d'un monomère faisant office d'emplacement de durcissement.

13. Procédé selon la revendication 5 dans lequel ledit fluoroélastomère est un copolymère de tétrafluoroéthylène et de perfluoro (alkylvinyléther) dans lequel le groupement alkyle est méthyle ou propyle.

14. Procédé selon la revendication 13 dans lequel ledit fluoroélastomère contient 0,1 à 5% en mole d'un monomère faisant office d'emplacement de durcissement.

15. Produit du procédé de la revendication 7.

16. Produit du procédé de la revendication 14.
